(19) Europäisches Patentamt
European Patent Office
Office européen des brevets

(11) EP 4 105 241 A1

(12) **EUROPEAN PATENT APPLICATION**
published in accordance with Art. 153(4) EPC

(43) Date of publication:
**21.12.2022 Bulletin 2022/51**

(21) Application number: **21775919.0**

(22) Date of filing: **26.03.2021**

(51) International Patent Classification (IPC):
***C08F 2/46*** *(2006.01)* ***B33Y 80/00*** *(2015.01)*
***B29C 64/129*** *(2017.01)* ***B29C 64/314*** *(2017.01)*
***A61K 6/15*** *(2020.01)* ***B33Y 70/00*** *(2020.01)*

(52) Cooperative Patent Classification (CPC):
**A61K 6/15; B29C 64/129; B29C 64/314; B33Y 70/00; B33Y 80/00; C08F 2/46**

(86) International application number:
**PCT/JP2021/013083**

(87) International publication number:
**WO 2021/193962 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2020 JP 2020056595**

(71) Applicant: **Mitsui Chemicals, Inc.**
**Tokyo 105-7122 (JP)**

(72) Inventors:
- **KIMURA, Mai**
  **Sodegaura-shi, Chiba 299-0265 (JP)**
- **SAKAMAKI, Toshikazu**
  **Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

(54) **PHOTOPOLYMERIZABLE COMPOSITION, THREE-DIMENSIONAL MOLDED ARTICLE, AND DENTAL PRODUCT**

(57) A photocurable composition, comprising a photopolymerizable component and a photopolymerization initiator, wherein: in a case in which a rectangular sheet-like test piece A1 with a length of 40 mm, a width of 10 mm, and a thickness of 1 mm, is produced by photomodeling under conditions in which the photocurable composition is irradiated with visible light having a wavelength of 405 nm at an irradiation dose of 12 mJ/cm$^2$ to form a cured layer A1 with a thickness of 50 μm, the cured layer A1 is stacked in a thickness direction thereof to form a rectangular sheet-like modeling product A1 with a length of 40 mm, a width of 10 mm, and a thickness of 1 mm, and the modeling product A1 is irradiated with ultraviolet rays having a wavelength of 365 nm at an irradiation dose of 10 J/cm$^2$ to produce the test piece A1, the test piece A1 has an X-ray absorption coefficient of from 9.0 cm$^{-1}$ to 34.0 cm$^{-1}$.

EP 4 105 241 A1

## Description

Technical Field

The present disclosure relates to a photocurable composition, a three-dimensional modeling product, and a dental product.

Background Art

Dental products such as dental prostheses and instruments for intraoral use have been studied in recent years. For example, in terms of the efficiency of modeling these dental products, methods of producing a three-dimensional modeling product such as a dental product by photomodeling using a 3D printer have been known (see, for example, Patent Document 1).

Patent Document 1: Japanese Patent No. 4160311

SUMMARY OF INVENTION

Technical Problem

However, in the production of a three-dimensional modeling product by photomodeling using a photocurable composition, a desired modeling accuracy cannot be obtained in some cases. For example, there are cases where a portion of the resulting three-dimensional modeling product has a thickness larger than a desired thickness in the propagation direction of light in the photomodeling (i.e., insufficient thickness accuracy in the propagation direction of light), or has a thickness larger than a desired thickness in a direction intersecting with (e.g., perpendicular to) the propagation direction of light in the photomodeling (i.e., insufficient thickness accuracy in a direction intersecting with (e.g., perpendicular to) the propagation direction of light).

Therefore, there is a demand for a photocurable composition from which a three-dimensional modeling product can be obtained with excellent modeling accuracy.

An object of one aspect of the disclosure is to provide: a photocurable composition from which a three-dimensional modeling product can be obtained with excellent modeling accuracy; a three-dimensional modeling product obtained from the photocurable composition; and a dental product.

Solution to Problem

Means for solving the above-described problems include the following aspects.

<1> A photocurable composition, comprising a photopolymerizable component and a photopolymerization initiator, wherein:
in a case in which a rectangular sheet-like test piece A1 with a length of 40 mm, a width of 10 mm, and a thickness of 1 mm, is produced by photomodeling under conditions in which the photocurable composition is irradiated with visible light having a wavelength of 405 nm at an irradiation dose of 12 mJ/$cm^2$ to form a cured layer A1 with a thickness of 50 $\mu$m, the cured layer A1 is stacked in a thickness direction thereof to form a rectangular sheet-like modeling product A1 with a length of 40 mm, a width of 10 mm, and a thickness of 1 mm, and the modeling product A1 is irradiated with ultraviolet rays having a wavelength of 365 nm at an irradiation dose of 10 J/$cm^2$ to produce the test piece A1, the test piece A1 has an X-ray absorption coefficient of from 9.0 $cm^{-1}$ to 34.0 $cm^{-1}$.
<2> The photocurable composition according to <1>, further comprising a filler.
<3> The photocurable composition according to <2>, wherein the filler is at least one selected from the group consisting of silica particles, zirconia particles, aluminosilicate particles, alumina particles, and titania particles.
<4> The photocurable composition according to <2> or <3>, wherein the filler has an average particle size of from 5 nm to 200 nm.
<5> The photocurable composition according to any one of <1> to <4>, wherein:
in a case in which a discoid test piece A2 with a diameter of 15 mm and a thickness of 1 mm, is produced by photomodeling under conditions in which the photocurable composition is irradiated with visible light having a wavelength of 405 nm at an irradiation dose of 12 mJ/$cm^2$ to form a cured layer A2 with a thickness of 100 $\mu$m, the cured layer A2 is stacked in a thickness direction thereof to form a discoid modeling product A2 with a diameter of 15 mm and a thickness of 1 mm, and the modeling product A2 is irradiated with ultraviolet rays having a wavelength of 365 nm at an irradiation dose of 10 J/$cm^2$ to produce the test piece A2, the test piece A2 has a Vickers hardness of 18 HV or more.

<6> The photocurable composition according to any one of claims 1 to 5, wherein:
in a case in which a rectangular rod-like test piece A3 with a length of 25 mm, a width of 2 mm, and a thickness of 2 mm, is produced by photomodeling under conditions in which the photocurable composition is irradiated with visible light having a wavelength of 405 nm at an irradiation dose of 12 $mJ/cm^2$ to form a cured layer A3 with a thickness of 100 μm, the cured layer A3 is stacked in a thickness direction thereof to form a rectangular rod-like modeling product A3 with a length of 25 mm, a width of 2 mm, and a thickness of 2 mm, and the modeling product A3 is irradiated with ultraviolet rays having a wavelength of 365 nm at an irradiation dose of 10 $J/cm^2$ to produce the test piece A3, the test piece A3 has a bending elastic modulus of 3,000 MPa or more.
<7> The photocurable composition according to any one of <1> to <6>, wherein the photopolymerizable component comprises a (meth)acrylic monomer.
<8> The photocurable composition according to <7>, wherein:

the (meth)acrylic monomer comprises at least one of a monofunctional (meth)acrylic monomer or a bifunctional (meth)acrylic monomer, and
a total amount of the monofunctional (meth)acrylic monomer and the bifunctional (meth)acrylic monomer is not less than 90% by mass with respect to a total amount of the (meth)acrylic monomer.

<9> The photocurable composition according to <7> or <8>, wherein the (meth)acrylic monomer comprises a bifunctional (meth)acrylic monomer.
<10> The photocurable composition according to any one of <1> to <9>, which is a photocurable composition for photomodeling.
<11> The photocurable composition according to any one of <1> to <10>, which is used for the production of a dental product by photomodeling.
<12> A three-dimensional modeling product, which is a cured product of the photocurable composition according to any one of <1> to <11>.
<13> A dental product, comprising the three-dimensional modeling product according to <12>.

Advantageous Effects of Invention

According to one aspect of the disclosure, the followings are provided: a photocurable composition from which a three-dimensional modeling product can be obtained with excellent modeling accuracy; a three-dimensional modeling product obtained from the photocurable composition; and a dental product.

BRIEF DESCRIPTION OF DRAWING

FIG. 1 is a schematic perspective view that illustrates one example of the three-dimensional modeling product according to the disclosure.

DESCRIPTION OF EMBODIMENTS

In the disclosure, those numerical ranges that are expressed with "to" each denote a range that includes the numerical values stated before and after "to" as the lower limit value and the upper limit value, respectively.
In the disclosure, the term "step" encompasses not only a discrete step but also a step that cannot be clearly distinguished from other steps, as long as the intended purpose of the step is achieved.
In the disclosure, when there are plural substances that correspond to a component of a composition, the indicated amount of the component in the composition means, unless otherwise specified, a total amount of the plural substances existing in the composition.
In a set of numerical ranges that are stated in a stepwise manner in the disclosure, the upper limit value or the lower limit value of one numerical range may be replaced with the upper limit value or the lower limit value of other numerical range. Further, in a numerical range stated in the disclosure, the upper limit value or the lower limit value of the numerical range may be replaced with a relevant value indicated in any of Examples.
In the disclosure, "light" is a concept that encompasses active energy rays such as ultraviolet rays and visible light beams.
In the disclosure, "(meth)acrylate" refers to an acrylate or a methacrylate, "(meth)acryloyl" refers to acryloyl or methacryloyl, and "(meth)acryl" refers to acryl or methacryl.

[Photocurable Composition]

The photocurable composition of the disclosure is a photocurable composition containing a photopolymerizable component and a photopolymerization initiator, wherein:
in a case in which a rectangular sheet-like test piece A1 with a length of 40 mm, a width of 10 mm, and a thickness of 1 mm, is produced by photomodeling under conditions in which the photocurable composition is irradiated with visible light having a wavelength of 405 nm at an irradiation dose of 12 $mJ/cm^2$ to form a cured layer A1 with a thickness of 50 $\mu m$, the cured layer A1 is stacked in a thickness direction thereof to form a rectangular sheet-like modeling product A1 with a length of 40 mm, a width of 10 mm, and a thickness of 1 mm, and the modeling product A1 is irradiated with ultraviolet rays having a wavelength of 365 nm at an irradiation dose of 10 $J/cm^2$ to produce the test piece A1, the test piece A1 has an X-ray absorption coefficient of from 9.0 $cm^{-1}$ to 34.0 $cm^{-1}$.

Conventionally, in the production of a three-dimensional modeling product by photomodeling using a photocurable composition, a desired modeling accuracy cannot be obtained in some cases. For example, there are cases where a portion of the resulting three-dimensional modeling product has a thickness larger than a desired thickness in the propagation direction of light in the photomodeling (i.e., insufficient thickness accuracy in the propagation direction of light), or has a thickness larger than a desired thickness in a direction intersecting with (e.g., perpendicular to) the propagation direction of light in the photomodeling (i.e., insufficient thickness accuracy in a direction intersecting with (e.g., perpendicular to) the propagation direction of light).

With regard to this point, according to the photocurable composition of the disclosure, a test piece A1, which is a cured product of the photocurable composition, has an X-ray absorption coefficient of from 9.0 $cm^{-1}$ to 34.0 $cm^{-1}$; therefore, a three-dimensional modeling product can be obtained from the photocurable composition with excellent modeling accuracy (e.g., excellent thickness accuracy).

One example of the above-described conventional problem and one example of the effects provided by the photocurable composition of the disclosure will now be described.

As photomodeling, vat photomodeling (i.e., photomodeling using a vat) is known.

In vat photomodeling, a photocurable composition (i.e., an uncured photocurable composition in a liquid state; the same applies below) housed in a vat is partially cured by photoirradiation to form a cured layer, and the cured layer is disposed on one another by repeating this operation, whereby a three-dimensional modeling product is obtained. Vat photomodeling is different from inkjet photomodeling in that it uses a vat.

Vat photomodeling is broadly classified into DLP (Digital Light Processing) photomodeling and SLA (Stereolithography) photomodeling.

In DLP photomodeling, a photocurable composition in a vat is irradiated with planar light. In SLA photomodeling, laser light is scanned over a photocurable composition in a vat.

In one example of DLP photomodeling, for example, a 3D printer (e.g., "CARA PRINT 4.0" manufactured by Kulzer GmbH, or "MAX UV" manufactured by Asiga) that includes the followings is employed:

a vertically movable build table;
a tray (i.e., a vat) which is arranged below the build table (on the side of the gravity direction; the same applies below) and which includes a light transmitting section and houses a photocurable composition; and
a light source (e.g., an LED light source) which is arranged below the tray and used for irradiating the photocurable composition in the tray with planar light through the light transmitting section of the tray.

In this example, first, a gap equivalent to a single layer is created between the build table and the tray, and this gap is filled with a photocurable composition. Next, the photocurable composition filling the gap is irradiated with planar light from below through the light transmitting section of the tray to cure the light-irradiated region, whereby a first cured layer is formed. Subsequently, the gap between the build table and the tray is expanded for another layer to be formed next, and the resulting space is filled with the photocurable composition. Then, the photocurable composition filling the space is irradiated with light in the same manner as in the curing of the first layer to form a second cured layer. The above-described operations are repeated to dispose cured layers on one another, whereby a three-dimensional modeling product is produced. In this example, the thus produced three-dimensional modeling product may be further irradiated with light and thereby further cured.

FIG. 1 is a schematic perspective view that illustrates one example of the three-dimensional modeling product according to the disclosure (three-dimensional modeling product 10).

As illustrated in FIG. 1, the three-dimensional modeling product 10 includes: a bottom portion 12; and a pair of side portions 14 and 16 facing each other. The pair of side portions 14 and 16 is substantially perpendicular to the bottom portion 12. A recess 20 is formed by the bottom portion 12 and the pair of side portions 14 and 16.

In FIG. 1, the z-direction means the propagation direction of light in the production process of the three-dimensional modeling product 10, the x-direction and the y-direction each mean a direction perpendicular to the z-direction,

and the x-direction and the y-direction are perpendicular to each other. The symbol "G" means the gravity direction.

The z-direction, which is the propagation direction of light, is opposite to the gravity direction G.

In the production of the three-dimensional modeling product 10 by DLP photomodeling, there is a case where, by disposing cured layers on one another, for example, the pair of side portions 14 and 16 is sequentially formed from the upper side (the opposite side of the gravity direction G) toward the lower side (the side of the gravity direction G), and the bottom portion 12 is formed in the end. At the completion of the formation of the bottom portion 12, the entirety of the resulting three-dimensional modeling product 10 is arranged between the build table and the tray, with the upper surface of the pair of side portions 14 and 16 being in contact with the build table.

In the step of forming the bottom portion 12, only a portion of a desired thickness in the photocurable composition arranged in the gap between the build table and the tray is cured to form a single cured layer, and this operation is repeated to dispose cured layers on one another, whereby the bottom portion 12 is formed. In other words, in the step of forming the cured layers constituting the bottom portion 12, the photocurable composition exists also in the region corresponding to the recess 20; however, the photocurable composition in this region corresponding to the recess 20 is not cured, and only the photocurable composition in the region corresponding to the cured layers constituting the bottom portion 12 is cured in the form of layers.

When a conventional photocurable composition is used, there is a case where, in the formation of the bottom portion 12, a single cured layer is excessively thicker than a desired thickness, as a result of which the thickness of the bottom portion 12 formed by disposing cured layers on one another may be excessively larger than a desired thickness (i.e., a set value).

It is noted here that the thickness of a cured layer and the thickness of the bottom portion 12 both mean the thickness in the propagation direction of light. The thickness of the bottom portion 12 may be hereinafter referred to as "z-direction thickness".

The reason why a single cured layer is excessively thicker than a desired thickness is believed to be because, due to an excessively high optical transparency of the photocurable composition, not only the portion of the photocurable composition that is required for the formation of the cured layer but also the portion that should not naturally be cured (i.e., the region corresponding to the recess 20) are cured.

Meanwhile, when a conventional photocurable composition is used and the irradiation dose of light is reduced so as to adjust a single cured layer to have a desired thickness, curing of the composition may be insufficient, and this may result in a modeling defect.

Further, when a conventional photocurable composition is used, there is also a case where, in the formation of the pair of side portions 14 and 16, the thickness of the pair of side portions 14 and 16 is excessively larger than a desired thickness (i.e., a set value).

It is noted here that the thickness of the pair of side portions 14 and 16 means the thickness in the x-direction that is perpendicular to the propagation direction of light (i.e., z-direction).

The direction of the thickness of the pair of side portions 14 and 16 may be hereinafter referred to as "x-direction thickness".

The reason why the x-direction thickness is larger than a desired thickness is believed to be because, due to an excessively low optical transparency of the photocurable composition, the light entering the photocurable composition is unlikely to propagate in the z-direction (i.e., natural propagation direction of light) and is instead more likely to be scattered in directions other than the z-direction (e.g., x-direction and y-direction). In other words, it is believed that, in the process of forming cured layers to form the pair of side portions 14 and 16, light scattered in directions other than the z-direction causes curing of even those parts that should not naturally be cured.

Meanwhile, when a conventional photocurable composition is used and the irradiation dose of light is reduced so as to inhibit such scattering of light, curing of the composition may be insufficient, and this may result in a modeling defect.

With regard to the above-described problems, when the photocurable composition of the disclosure is used, not only the phenomenon that the thickness of the resulting bottom portion 12 is excessively large can be inhibited (i.e., the thickness accuracy in the z-direction can be improved), but also the phenomenon that the thickness of the pair of side portions 14 and 16 is excessively large can be inhibited (i.e., the thickness accuracy in the x-direction can be improved).

The reason why this effect is exerted is believed to be because, since the test piece A1, which is a cured product of the photocurable composition of the disclosure, has an X-ray absorption coefficient of from 9.0 $cm^{-1}$ to 34.0 $cm^{-1}$, excessive and insufficient optical transparency of the photocurable composition are inhibited.

Specifically, an excessive optical transparency of the photocurable composition is inhibited when the test piece A1 has an X-ray absorption coefficient of 9.0 $cm^{-1}$ or more. As a result, the thickness accuracy in the propagation direction of light in photomodeling (z-direction in the above-described example) is improved.

An insufficient optical transparency of the photocurable composition is inhibited when the test piece A1 has an X-ray absorption coefficient of 34.0 $cm^{-1}$ or less. By this, scattering of light in directions other than the propagation

direction of light in photomodeling (x-direction in the above-described example) is inhibited, as a result of which the thickness accuracy in directions other than the propagation direction of light in photomodeling is improved.

As described above, the feature that the test piece A1 has an X-ray absorption coefficient of from 9.0 $cm^{-1}$ to 34.0 $cm^{-1}$ means that the optical transparency of the photocurable composition of the disclosure is neither excessively high nor excessively low and is within a specific range.

In the disclosure, in order to strictly specify a range of the optical transparency of the photocurable composition, not the transmittance of the photocurable composition itself, but a range of the X-ray absorption coefficient of the test piece A1, which is a cured product of the photocurable composition, is specified.

In other words, the X-ray absorption coefficient of the test piece A1 in the disclosure is an index of the transparency of the photocurable composition of the disclosure. Accordingly, the conditions for the production of a three-dimensional modeling product using the photocurable composition of the disclosure do not necessarily have to be the same as the conditions for the production of the test piece A1.

The above-described problems in the thickness accuracy of the three-dimensional modeling product 10 are not limited to the three-dimensional modeling product 10 and can generally occur in those three-dimensional products that have at least one of a recess and a space (e.g., dental products).

It is noted here that the concept of a recess encompasses a recess formed by a bottom portion and a pair of side portions (e.g., recess 20), a bottomed hole, and the like.

Further, the concept of a space encompasses an internal space completely surrounded by walls of a three-dimensional modeling product, a through-hole, and the like.

According to the photocurable composition of the disclosure, the modeling accuracy can be improved not only in the case of producing the three-dimensional modeling product 10, but also in the case of producing a general three-dimensional modeling product.

<Use>

The use of the photocurable composition of the disclosure is not particularly limited.

From the standpoint of allowing the effect of improving the modeling accuracy of a three-dimensional modeling product to be more effectively exerted, the photocurable composition of the disclosure is preferably a photocurable composition for photomodeling.

From the standpoint of allowing the effect of improving the thickness accuracy of a three-dimensional modeling product to be more effectively exerted, the photocurable composition of the disclosure is more preferably a photocurable composition for vat photomodeling (e.g., DLP or SLA photomodeling, preferably DLP photomodeling).

Moreover, from the standpoint of allowing the effect of improving the modeling accuracy of a three-dimensional modeling product to be more effectively exerted, the photocurable composition of the disclosure is preferably a photocurable composition used for the production of a dental product.

The dental product is, for example, a denture (i.e., an artificial tooth), a denture base, a dental prosthesis, a medical instrument for intraoral use, a dental model, or a lost-foam casting model.

Examples of the dental prosthesis include inlays, crowns, bridges, temporary crowns, and temporary bridges.

Examples of the medical instrument for intraoral use include mouthpieces, mouthguards, orthodontic appliances, bite splints, impression trays, and surgical guides.

Examples of the dental model include jaw models.

<X-ray Absorption Coefficient of Test Piece A1>

As described above, the test piece A1, which is a cured product of the photocurable composition of the disclosure, has an X-ray absorption coefficient of from 9.0 $cm^{-1}$ to 34.0 $cm^{-1}$.

As described above, when the X-ray absorption coefficient of the test piece A1 is 9.0 $cm^{-1}$ or more, the thickness accuracy in the propagation direction of light in photomodeling (the z-direction in the above-described example) is improved.

As described above, when the X-ray absorption coefficient of the test piece A1 is 34.0 $cm^{-1}$ or less, the thickness accuracy in directions other than the propagation direction of light in photomodeling (the x-direction in the above-described example) is improved.

The X-ray absorption coefficient of the test piece A1 is preferably from 9.0 $cm^{-1}$ to 32.0 $cm^{-1}$, more preferably from 10.0 $cm^{-1}$ to 31.0 $cm^{-1}$, still more preferably from 12.0 $cm^{-1}$ to 30.0 $cm^{-1}$, particularly preferably from 20.0 $cm^{-1}$ to 30.0 $cm^{-1}$.

(Test Piece A1)

The test piece A1 is a rectangular sheet-like test piece of 40 mm in length, 10 mm in width, and 1 mm in thickness.

The test piece A1 is produced by photomodeling under conditions in which the photocurable composition is irradiated with visible light having a wavelength of 405 nm at an irradiation dose of 12 mJ/cm$^2$ to form a cured layer A1 with a thickness of 50 $\mu$m, the cured layer A1 is stacked in a thickness direction thereof to form a rectangular sheet-like modeling product A1 with a length of 40 mm, a width of 10 mm, and a thickness of 1 mm, and the modeling product A1 is irradiated with ultraviolet rays having a wavelength of 365 nm at an irradiation dose of 10 J/cm$^2$ to produce the test piece A1.

The test piece A1 can be produced, for example, in accordance with the above-described example of DLP photomodeling.

In the below-described Examples, the test piece A1 was produced using "CARA PRINT 4.0" manufactured by Kulzer GmbH, which is a DLP-type 3D printer.

(X-ray Absorption Coefficient)

The X-ray absorption coefficient of the test piece A1 is determined as follows using a small-angle X-ray scattering apparatus (SAXS).

An X-ray direct beam emitted from an X-ray source is attenuated by a semi-transparent beam stopper, and the thus attenuated X-ray is allowed to pass through the test piece A1. Under this condition, the intensity ($I_0$) of the X-ray before passing through the test piece A1 and the intensity (I) of the X-ray that has passed through the test piece A1 are each measured and, based on the thus obtained $I_0$ and I values and the thickness of the test piece A1 (t; i.e. 1 mm), the X-ray absorption coefficient (cm$^{-1}$) is determined using the following equation:

$$\text{X-ray absorption coefficient (cm}^{-1}\text{)} = (-\ln(I/I_0))/t$$

With regard to the X-ray absorption coefficient of the test piece A1 in the below-described Examples, the small-angle X-ray scattering apparatus (SAXS) and the measurement conditions were as follows.

Apparatus: NanoViewer, manufactured by Rigaku Corporation
X-ray source: CuK$\alpha$
Output: 40 kV, 30 mA
Detector: PILATUS 100K

<Vickers Hardness of Test Piece A2>

In a case in which a discoid test piece A2 with a diameter of 15 mm and a thickness of 1 mm, is produced by photomodeling under conditions in which the photocurable composition is irradiated with visible light having a wavelength of 405 nm at an irradiation dose of 12 mJ/cm$^2$ to form a cured layer A2 with a thickness of 100 $\mu$m, the cured layer A2 is stacked in a thickness direction thereof to form a discoid modeling product A2 with a diameter of 15 mm and a thickness of 1 mm, and the modeling product A2 is irradiated with ultraviolet rays having a wavelength of 365 nm at an irradiation dose of 10 J/cm$^2$ to produce the test piece A2, the test piece A2 preferably has a Vickers hardness of 18 HV or more.

When the Vickers hardness of the test piece A2 is 18 HV or more, a three-dimensional modeling product (e.g., a dental product) produced from the photocurable composition of the disclosure has superior hardness.

The Vickers hardness of the test piece A2 is more preferably 20 HV or more.

An upper limit of the Vickers hardness of the test piece A2 is not particularly limited; however, it is, for example, 30 HV or 26 HV

It is noted here that the conditions for the production of a three-dimensional modeling product using the photocurable composition of the disclosure do not necessarily have to be the same as the conditions for the production of the test piece A2. Even when the conditions for the production of a three-dimensional modeling product are different from the conditions for the production of the test piece A2, there is a correlation between the Vickers hardness of the test piece A2 and the hardness of the three-dimensional modeling product.

In other words, the Vickers hardness of the test piece A2 is an index of the hardness of a three-dimensional modeling product produced from the photocurable composition of the disclosure.

(Test Piece A2)

The test piece A2 can be produced, for example, in accordance with the above-described example of DLP photomodeling.
In the below-described Examples, the test piece A2 was produced using "CARA PRINT 4.0" manufactured by Kulzer GmbH, which is a DLP-type 3D printer.

(Vickers Hardness)

The Vickers hardness of the test piece A2 is measured in accordance with JIS T6517:2011.
Specifically, the test piece A2 is immersed in purified water at 37 ± 1°C for 24 ± 2 hours.
Subsequently, the test piece A2 is recovered from purified water, and the Vickers hardness of the light-irradiated surface (i.e., the surface of the side irradiated with light at the time of production) of the recovered test piece A2 is measured in accordance with the Vickers hardness test method prescribed in JIS Z2244 at a test force of 200 g. The thus obtained value is defined as the Vickers hardness of the test piece A2.
In the below-described Examples, a micro hardness tester HMV-G (manufactured by Shimadzu Corporation) was employed as a Vickers hardness measuring device.

<Bending elastic modulus of Test Piece A3>

In a case in which a rectangular rod-like test piece A3 with a length of 25 mm, a width of 2 mm, and a thickness of 2 mm, is produced by photomodeling under conditions in which the photocurable composition is irradiated with visible light having a wavelength of 405 nm at an irradiation dose of 12 $mJ/cm^2$ to form a cured layer A3 with a thickness of 100 μm, the cured layer A3 is stacked in a thickness direction thereof to form a rectangular rod-like modeling product A3 with a length of 25 mm, a width of 2 mm, and a thickness of 2 mm, and the modeling product A3 is irradiated with ultraviolet rays having a wavelength of 365 nm at an irradiation dose of 10 $J/cm^2$ to produce the test piece A3, the test piece A3 preferably has a bending elastic modulus of 3,000 MPa or more.
When the bending elastic modulus of the test piece A3 is 3,000 MPa or more, a three-dimensional modeling product (e.g., a dental product) produced from the photocurable composition of the disclosure has superior bending elastic modulus.
The bending elastic modulus of the test piece A3 is more preferably 4,000 MPa or more.
An upper limit of the bending elastic modulus of the test piece A3 is not particularly limited; however, it is, for example, 6,000 MPa.
It is noted here that the conditions for the production of a three-dimensional modeling product using the photocurable composition of the disclosure do not necessarily have to be the same as the conditions for the production of the test piece A3. Even when the conditions for the production of a three-dimensional modeling product are different from the conditions for the production of the test piece A3, there is a correlation between the bending elastic modulus of the test piece A3 and the bending elastic modulus of the three-dimensional modeling product.
In other words, the bending elastic modulus of the test piece A3 is an index of the bending elastic modulus of a three-dimensional modeling product produced from the photocurable composition of the disclosure.

(Test Piece A3)

The test piece A3 can be produced, for example, in accordance with the above-described example of DLP photomodeling.
In the below-described Examples, the test piece A3 was produced using "CARA PRINT 4.0" manufactured by Kulzer GmbH, which is a DLP-type 3D printer.

(Bending elastic modulus)

The bending elastic modulus of the test piece A3 is measured as follows.
The test piece A3 is immersed in purified water at 37 ± 1°C for 24 ± 2 hours.
Subsequently, the test piece A3 is recovered from purified water, and the bending elastic modulus of the recovered test piece A3 is measured in accordance with ISO10477:2004 at a test speed of 1 ± 0.3 mm/min.
In the below-described Examples, a universal tester (manufactured by INTESCO Co., Ltd.) was employed as a bending elastic modulus measuring device.

<Bending Strength of Test Piece A3>

When the above-described test piece A3 is produced from the photocurable composition of the disclosure, the test piece A3 preferably has a bending strength of 110 MPa or more.

When the bending strength of the test piece A3 is 110 MPa or more, a three-dimensional modeling product (e.g., a dental product) produced from the photocurable composition of the disclosure has superior bending strength.

The bending strength of the test piece A3 is more preferably 120 MPa or more, still more preferably 125 MPa or more, yet still more preferably 130 MPa or more.

An upper limit of the bending strength of the test piece A3 is not particularly limited; however, it is, for example, 170 MPa, 160 MPa, or 150 MPa.

It is noted here that the conditions for the production of a three-dimensional modeling product using the photocurable composition of the disclosure do not necessarily have to be the same as the conditions for the production of the test piece A3. Even when the conditions for the production of a three-dimensional modeling product are different from the conditions for the production of the test piece A3, there is a correlation between the bending strength of the test piece A3 and the bending strength of the three-dimensional modeling product.

In other words, the bending strength of the test piece A3 is an index of the bending strength of a three-dimensional modeling product produced from the photocurable composition of the disclosure.

(Bending strength)

The bending strength of the test piece A3 is measured as follows.

The test piece A3 is immersed in purified water at 37 ± 1°C for 24 ± 2 hours.

Subsequently, the test piece A3 is recovered from purified water, and the bending strength of the recovered test piece A3 is measured in accordance with ISO10477:2004 at a test speed of 1 ± 0.3 mm/min.

In the below-described Examples, a universal tester (manufactured by INTESCO Co., Ltd.) was employed as a bending strength measuring device.

(Photopolymerizable Component)

The photocurable composition of the disclosure contains at least one kind of photopolymerizable component.

The photopolymerizable component is, for example, a compound containing an ethylenic double bond.

Examples of the compound containing an ethylenic double bond include (meth)acrylic monomers, styrene, styrene derivatives, and (meth)acrylonitrile.

As the photopolymerizable component, any of the photopolymerizable components described in the paragraphs [0030] to [0059] of WO 2019/189652 may be used as well.

The photopolymerizable component preferably contains at least one kind of (meth)acrylic monomer.

In this case, a total ratio of the (meth)acrylic monomer with respect to the whole photopolymerizable component is preferably not less than 80% by mass, more preferably not less than 90% by mass, still more preferably not less than 95% by mass.

The (meth)acrylic monomer may be any monomer as long as it contains one or more (meth)acryloyl groups in the molecule, and there is no other particular limitation.

The (meth)acrylic monomer may be a monofunctional (meth)acrylic monomer (i.e., a monomer having one (meth)acryloyl group in the molecule), a bifunctional (meth)acrylic monomer (i.e., a monomer having two (meth)acryloyl groups in the molecule), or a polyfunctional (meth)acrylic monomer (i.e., a monomer having three or more (meth)acryloyl groups in the molecule).

The (meth)acrylic monomer preferably contains at least one of an aromatic structure (e.g., a bisphenol A structure), an alicyclic structure, and a urethane bond in the molecule.

The (meth)acrylic monomer of this preferred aspect may further contain at least one of an ethyleneoxy group or a propyleneoxy group.

The molecular weight of the (meth)acrylic monomer is preferably 5,000 or less, more preferably 3,000 or less, still more preferably 2,000 or less, yet still more preferably 1,500 or less, further more preferably 1,000 or less, still further more preferably 800 or less.

A lower limit of the molecular weight of the (meth)acrylic monomer is not particularly limited as long as the (meth)acrylic monomer is a monomer that contains one or more (meth)acryloyl groups in the molecule. The lower limit of the molecular weight of the (meth)acrylic monomer is, for example, 86, preferably 100, more preferably 200, still more preferably 300.

From the standpoint of reducing the viscosity of the photocurable composition, the (meth)acrylic monomer that may be contained in the photocurable composition of the disclosure preferably contains at least one of a monofunctional

(meth)acrylic monomer or a bifunctional (meth)acrylic monomer.

In this case, from the standpoint of reducing the viscosity of the photocurable composition, a total amount of the monofunctional (meth)acrylic monomer and the bifunctional (meth)acrylic monomer is preferably not less than 60% by mass, more preferably not less than 80% by mass, still more preferably not less than 90% by mass, with respect to a total amount of the (meth)acrylic monomer that may be contained in the photocurable composition of the disclosure.

Specific examples of the monofunctional (meth)acrylic monomer include cyclohexyl (meth)acrylate, isobornyl (meth)acrylate, dicyclopentanyl (meth)acrylate, dicyclopentenyl (meth)acrylate, 4-tert-butylcyclohexyl (meth)acrylate, tetrahydrofurfuryl (meth)acrylate, (2-methyl-2-ethyl-1,3-dioxolan-4-yl)methyl (meth)acrylate, cyclic trimethylolpropane formal (meth)acrylate, 4-(meth)acryloyl morpholine, lauryl (meth)acrylate, 4-hydroxybutyl (meth)acrylate, 2-hydroxyethyl (meth)acrylate, benzyl (meth)acrylate, 2-phenoxyethyl (meth)acrylate, 3-phenoxybenzyl (meth)acrylate, 2-hydroxy-3-phenoxypropyl (meth)acrylate, phenoxyethylene glycol (meth)acrylate, 2-dodecyl-1-hexadecanyl (meth)acrylate, 2-(meth)acryloyloxyethyl succinate, 2-[[(butylamino)carbonyl]oxy]ethyl (meth)acrylate, and 2-(2-ethoxyethoxy)ethyl (meth)acrylate.

Specific examples of the bifunctional (meth)acrylic monomer include ethylene glycol di(meth)acrylate, triethylene glycol di(meth)acrylate, glycerol di(meth)acrylate, 1,6-hexanediol di(meth)acrylate, ethoxylated bisphenol A di(meth)acrylate, dimethylol-tricyclodecane di(meth)acrylate, 1,9-nonanediol di(meth)acrylate, dioxane glycol di(meth)acrylate, diethylene glycol di(meth)acrylate, dipropylene glycol di(meth)acrylate, ethoxylated bisphenol A di(meth)acrylate, ethoxylated hydrogenated bisphenol A di(meth)acrylate, 2-hydroxy-3-acryloyloxypropyl (meth)acrylate, bis(2-(meth)acryloxyethyl)-N,N'-1,9-nonylene biscarbamate (diurethane (meth)acrylate), polyethylene glycol di(meth)acrylate, and polypropylene glycol di(meth)acrylate.

From the standpoint of further facilitating the curing of the photocurable composition and further improving the modeling accuracy of a three-dimensional modeling product, the (meth)acrylic monomer that may be contained in the photocurable composition of the disclosure preferably contains a bifunctional (meth)acrylic monomer.

In this case, from the standpoint of further facilitating the curing of the photocurable composition and further improving the modeling accuracy of a modeling product, a total amount of the bifunctional (meth)acrylic monomer is preferably not less than 30% by mass, more preferably not less than 40% by mass, still more preferably not less than 50% by mass, with respect to a total amount of the (meth)acrylic monomer that may be contained in the photocurable composition of the disclosure.

From the standpoint of further facilitating the curing of the photocurable composition and further improving the modeling accuracy of a modeling product, the (meth)acrylic monomer that may be contained in the photocurable composition of the disclosure more preferably contains:

a monomer M1, which is a bifunctional (meth)acrylic monomer containing at least one of an aromatic ring structure (e.g., a bisphenol A structure) or an alicyclic structure in the molecule; and/or
a monomer M2, which is a bifunctional (meth)acrylic monomer containing a urethane bond in the molecule.
In this case, a total amount of the monomer M1 and the monomer M2 is preferably not less than 30% by mass, more preferably not less than 40% by mass, still more preferably not less than 50% by mass, with respect to a total amount of the (meth)acrylic monomer that may be contained in the photocurable composition of the disclosure.

The amount of the photopolymerizable component contained in the photocurable composition of the disclosure is not particularly limited.

From the standpoint of further improving the modeling accuracy of a three-dimensional modeling product, the content of the photopolymerizable component is preferably not less than 40 parts by mass, more preferably not less than 50 parts by mass, still more preferably not less than 60 parts by mass, with respect to 100 parts by mass of the photocurable composition.

(Photopolymerization Initiator)

The photocurable composition of the disclosure contains at least one kind of photopolymerization initiator.

Examples of the photopolymerization initiator include alkylphenone compounds, acylphosphine oxide compounds, titanocene compounds, oxime ester compounds, benzoin compounds, acetophenone compounds, benzophenone compounds, thioxanthone compounds, $\alpha$-acyloxime ester compounds, phenyl glyoxylate compounds, benzyl compounds, azo compounds, diphenyl sulfide compounds, iron-phthalocyanine compounds, benzoin ether compounds, and anthraquinone compounds.

From the standpoint of the reactivity, the photopolymerization initiator preferably contains at least one selected from the group consisting of alkylphenone compounds and acylphosphine oxide compounds.

From the standpoint of improving the modeling accuracy of a three-dimensional modeling product, the photopolymerization initiator preferably contains an acylphosphine oxide compound (e.g., 2,4,6-trimethylbenzoyl-diphenyl-phosphine oxide or bis(2,4,6-trimethylbenzoyl)phenyl phosphine oxide), more preferably contains 2,4,6-trimethylbenzoyl-diphenyl-phosphine oxide.

The amount of the photopolymerization initiator contained in the photocurable composition of the disclosure is preferably from 0.1 parts by mass to 20 parts by mass, more preferably from 0.2 parts by mass to 10 parts by mass, still more preferably from 0.3 parts by mass to 5 parts by mass, yet still more preferably from 0.3 parts by mass to 3 parts by mass, with respect to 100 parts by mass of the photopolymerizable component.

(Filler)

The photocurable composition of the disclosure preferably further contains at least one kind of filler.

When the photocurable composition of the disclosure contains a filler, the X-ray absorption coefficient of the test piece A1 is more likely to be achieved in the above-described range.

The filler is preferably inorganic particles, more preferably inorganic oxide particles.

The filler is still more preferably at least one selected from the group consisting of silica particles (i.e., silicon oxide particles), zirconia particles (i.e., zirconium oxide particles), aluminosilicate particles, alumina particles (i.e., aluminum oxide particles), and titania particles (i.e., titanium oxide particles).

The filler particularly preferably contains silica particles.

The average particle size of the filler is not particularly limited; however, from the standpoint of making the X-ray absorption coefficient of the test piece A1 more likely to be achieved in the above-described range, the average particle size of the filler is preferably from 5 nm to 500 nm, more preferably from 5 nm to 200 nm, still more preferably from 5 nm to 100 nm, yet still more preferably from 5 nm to 70 nm.

From the standpoint of improving the wear resistance, the average particle size of the filler is preferably 40 nm or larger, more preferably 50 nm or larger, still more preferably 60 nm or larger, particularly preferably 70 nm or larger.

In the disclosure, the average particle size of the filler means the number-average primary particle size, specifically a value determined as follows.

A cured product (e.g., the above-described test piece A1) of the photocurable composition of the disclosure is obtained by photomodeling, and a cross-section is subsequently cut out from the cured product. A TEM image of the thus obtained cross-section is taken, and 100 particles are randomly selected. The circle equivalent diameters of these particles are determined, and the arithmetic mean (number-average) of the thus determined circle equivalent diameters is calculated.

When the photocurable composition of the disclosure contains a filler, the content of the filler is preferably from 2 parts by mass to 100 parts by mass, more preferably from 5 parts by mass to 80 parts by mass, still more preferably from 5 parts by mass to 60 parts by mass, yet still more preferably from 10 parts by mass to 50 parts by mass, with respect to 100 parts by mass of the photopolymerizable component.

Depending on the intended purpose, the filler may be surface-treated with a surface treatment agent such as a silane coupling agent. By the surface treatment agent, for example, wear resistance can be imparted to a cured product of the photocurable composition containing the filler.

The surface treatment agent is not particularly limited and, for example, a silane coupling agent can be used.

Examples of the silane coupling agent include organosilicon compounds, such as methacryloxyalkyltrimethoxysilane (number of carbon atoms between a methacryloxy group and a silicon atom: from 3 to 12), methacryloxyalkyltriethoxysilane (number of carbon atoms between a methacryloxy group and a silicon atom: from 3 to 12), vinyltrimethoxysilane, vinylethoxysilane, and vinyltriacetoxysilane.

(Other Components)

If necessary, the photocurable composition of the disclosure may also contain other components in addition to the above-described ones.

Examples of the other components include color materials, modifiers, stabilizers, antioxidants, and solvents.

<Preferred Viscosity of Photocurable Composition>

The photocurable composition of the disclosure has a viscosity, which is measured by an E-type viscometer under the conditions of 25°C and 50 rpm (hereinafter, also simply referred to as "viscosity"), of preferably from 5 mPa·s to 6,000 mPa·s.

It is noted here that "rpm" means revolutions per minute (rotations per minute).

When the viscosity is from 5 mPa·s to 6,000 mPa·s, the photocurable composition has excellent ease of handling in the production of a three-dimensional modeling product by photomodeling.

The viscosity is more preferably from 10 mPa·s to 5,000 mPa·s, still more preferably from 20 mPa·s to 4,000

mPa·s, yet still more preferably from 100 mPa·s to 3,000 mPa·s, further more preferably from 200 mPa·s to 2,000 mPa·s, still further more preferably from 400 mPa·s to 1,500 mPa·s.

[Three-Dimensional Modeling product]

The three-dimensional modeling product of the disclosure is a cured product of the above-described photocurable composition of the disclosure.
Therefore, the three-dimensional modeling product of the disclosure has excellent modeling accuracy.
The three-dimensional modeling product of the disclosure is preferably a three-dimensional modeling product having at least one of a recess or a space.
The recess and the space are as described above.

[Dental Product]

The dental product of the disclosure includes the above-described three-dimensional modeling product of the disclosure (preferably a three-dimensional modeling product having a recess or a space).
Therefore, the dental product of the disclosure has excellent modeling accuracy.
Specific examples of the dental product are as described above.

EXAMPLES

Examples of the disclosure will now be described; however, the disclosure is not limited to the below-described Examples.

<Production of Photocurable Compositions>

Photocurable compositions of Examples 1 to 5 and Comparative Examples 1 and 2 were each prepared by kneading the materials shown in Table 1 below using a three-roll mill.
The details of the materials (photopolymerizable components, photopolymerization initiators, and fillers) shown in Table 1 below are as follows.
In Table 1 below, the numerical values shown in the columns of the respective components used in Examples and Comparative Examples each indicate the amount (parts by mass) of each component with respect to a total of 100 parts by mass of photopolymerizable components.

(Photopolymerizable Components)

EBECRYL 4859: urethane dimethacrylate (manufactured by Daicel-Allnex Ltd.; the structure is shown below)

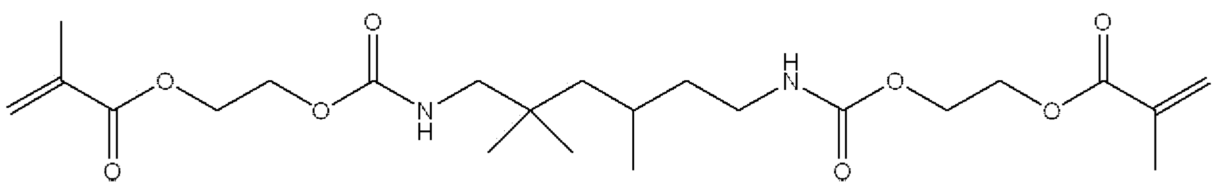

POBA: 3-phenoxybenzyl acrylate (manufactured by Kyoeisha Chemical Co., Ltd.; the structure is shown below)

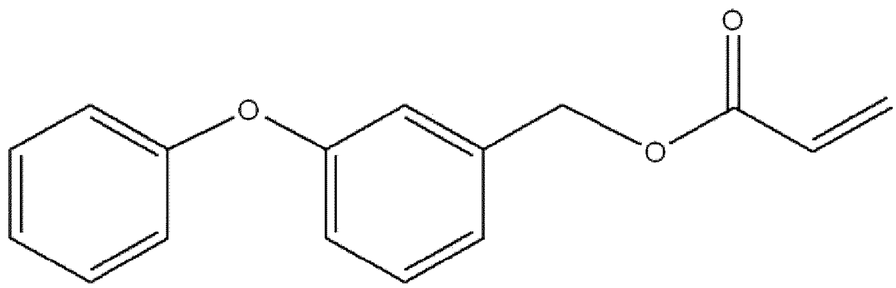

M600A: 2-hydroxy-3-phenoxypropyl acrylate (manufactured by Kyoeisha Chemical Co., Ltd.; the structure is shown below)

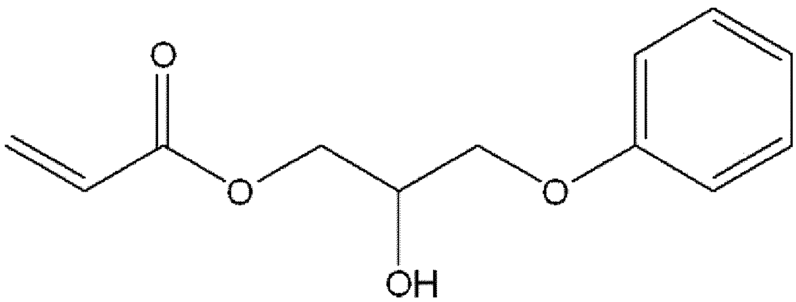

SR348: ethoxylated bisphenol A dimethacrylate (manufactured by Arkema K.K.; the structure is shown below)

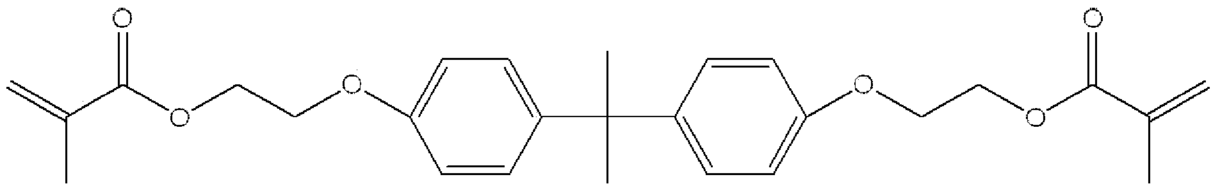

SR340: 2-phenoxyethyl methacrylate (manufactured by Arkema K.K.; the structure is shown below)

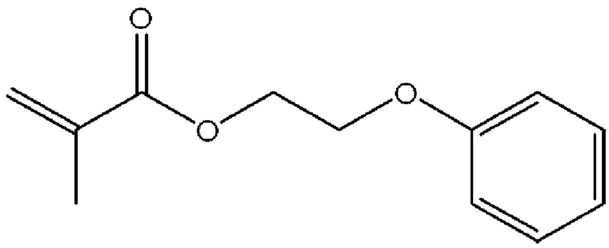

(Photopolymerization Initiators)

TPO: acylphosphine oxide compound (specifically 2,4,6-trimethylbenzoyl-diphenyl-phosphine oxide) (OMNIRAD TPO H, manufactured by IGM Resins B.V; the structure is shown below)

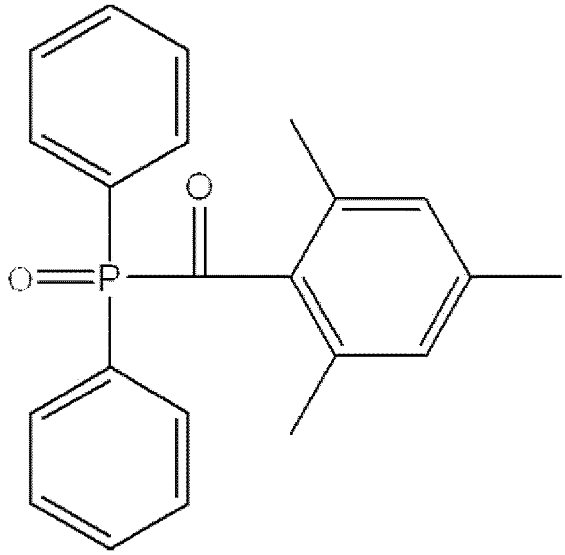

819: acylphosphine oxide compound (specifically bis(2,4,6-trimethylbenzoyl)phenyl phosphine oxide) (OMNIRAD 819, manufactured by IGM Resins B.V; the structure is shown below)

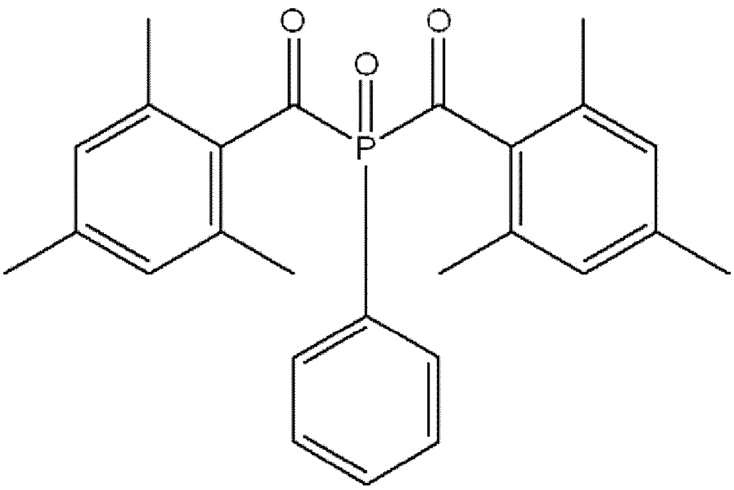

(Fillers)

SC4500-SMJ (silica particles, average particle size: 1,000 nm, manufactured by Admatechs Co., Ltd.)
AEROSIL #200 (silica particles, average particle size: 12 nm, manufactured by Nippon Aerosil Co., Ltd.)
AEROSIL #90G (silica particles, average particle size: 22 nm, manufactured by Nippon Aerosil Co., Ltd.)
ADMANANO YA050C-SM1 (silica particles, average particle size: 50 nm, manufactured by Admatechs Co., Ltd.)
AEROSIL #R972 (silica, average particle size: 16 nm, manufactured by Nippon Aerosil Co., Ltd.)
ADMANANO YC100C-SM1 (silica particles, average particle size: 100 nm, manufactured by Admatechs Co., Ltd.)

[Examples 1 to 5 and Comparative Examples 1 and 2]

<X-ray Absorption Coefficient of Test Piece A1>

A rectangular sheet-like test piece A1 was produced by the above-described method using each photocurable composition of Examples and Comparative Examples, and the X-ray absorption coefficient ($cm^{-1}$) of the thus produced test piece A1 was measured.
The results thereof are shown in Table 1.

<Vickers Hardness of Test Piece A2>

The above-described discoid test piece A2 was produced by the above-described method using each photocurable composition of Examples and Comparative Examples, and the Vickers hardness (HV) of the thus produced test piece A2 was measured.
The results thereof are shown in Table 1.

<Bending elastic modulus of Test Piece A3>

The above-described rectangular rod-like test piece A3 was produced by the above-described method using each photocurable composition of Examples and Comparative Examples, and the bending elastic modulus (MPa) of the thus produced test piece A3 was measured.
The results thereof are shown in Table 1.

<Bending strength of Test Piece A3>

The above-described rectangular rod-like test piece A3 was produced by the above-described method using each photocurable composition of Examples and Comparative Examples, and the bending strength (MPa) of the thus produced test piece A3 was measured.
The results thereof are shown in Table 1.

<Evaluation of Thickness Accuracy of Three-Dimensional Modeling product>

Using a DLP-type 3D printer (CARA PRINT 4.0, manufactured by Kulzer GmbH), the above-described three-dimensional modeling product 10 illustrated in FIG. 1 was produced by DLP photomodeling.
In the thus produced three-dimensional modeling product 10, the bottom portion 12 had a thickness (design value) of 1.00 mm, and the side portion 14 had a thickness (design value) of 1.60 mm.
In the production of the three-dimensional modeling product 10, as in the above-described example, the side portions 14 and 16 were sequentially formed from the upper side (the opposite side of the gravity direction G) toward the lower side (the side of the gravity direction G) of the three-dimensional modeling product 10, and the bottom portion 12 was formed in the end. The detailed operations are as in the above-described example.
Each cured layer was formed by irradiating the photocurable composition with visible light having a wavelength of 405 nm at an irradiation dose of 12 $mJ/cm^2$, whereby a modeling product was obtained. The thickness of each cured layer was set at 50 $\mu$m. The thus obtained modeling product was irradiated with ultraviolet light having a wavelength of 365 nm at an irradiation dose of 10 $J/cm^2$ to produce the three-dimensional modeling product 10.
For the thus produced three-dimensional modeling product 10, the thickness of the bottom portion 12 and that of the side portion 14 were each measured using calipers (CD-P15S, manufactured by Mitutoyo Corporation).
In Table 1, the measured value of the thickness of the bottom portion 12 is shown as "z-direction thickness (mm)", and the measured value of the thickness of the side portion 14 is shown as "x-direction thickness (mm)".
Further, for each of the measured value of the thickness of the bottom portion 12 and the measured value of the thickness of the side portion 14, the deviation (%) from the respective design value was determined using the following equation to evaluate the thickness accuracy based on the below-described evaluation criteria.
Deviation from design value (%) = ((Measured value - Design value)/Design value) $\times$ 100
The results are shown in Table 1.
In the following evaluation criteria, "A" is the most excellent rank of the thickness accuracy.

-Evaluation Criteria of Thickness Accuracy-

A: The absolute value of the deviation (%) from the design value was 15% or less.
B: The absolute value of the deviation (%) from the design value was 15% or more but 30% or less.
C: The absolute value of the deviation (%) from the design value was more than 30%.

[Table 1]

| Components of photocurable composition | | Average particle size (nm) | Comparative Example 1 | Comparative Example 2 | Example 1 | Example 2 | Example 3 | Example 4 | Example 5 |
|---|---|---|---|---|---|---|---|---|---|
| Filler | SC4500-SMJ | 1,000 | | 40 | | | | | |
| | AEROSIL #200 | 12 | | | 35 | | | | |
| | AEROSIL#90G | 22 | | | | 40 | | | |
| | ADMANANO YA050C-SM1 | 50 | | | | | 40 | | |
| | AEROSIL #R972 | 16 | | | | | | 15 | |
| | ADMANANO YC100C-SM1 | 100 | | | | | | | 40 |
| Photopolymerizable component | EBECRYL 4859 | - | 60 | 60 | 60 | 60 | 60 | | 60 |
| | POBA | - | 20 | 20 | 20 | 20 | 20 | | 20 |
| | M600A | - | 20 | 20 | 20 | 20 | 20 | | 20 |
| | SR348 | - | | | | | | 80 | |
| | SR340 | - | | | | | | 20 | |
| Photopolymerization initiator | TPO | - | 1 | 1 | 1 | 1 | 1 | | 1 |
| | 819 | - | | | | | | 1 | |
| Total amount | | | 101 | 141 | 136 | 141 | 141 | 116 | 141 |
| X-ray absorption coefficient of test piece A1 ($cm^{-1}$) | | | 7.5 | 36.1 | 24.1 | 28.3 | 24.7 | 13.6 | 28.1 |
| Vickers hardness of test piece A2 (HV) | | | 15 | 23 | 24 | 24 | 24 | 22 | 25 |
| Bending strength of test piece A3 (MPa) | | | 102 | 115 | 141 | 143 | 132 | 149 | 137 |
| Bending elastic modulus of test piece A3 (MPa) | | | 2,568 | 4,106 | 4,899 | 5,563 | 4,979 | 4,210 | 5,319 |

(continued)

| Components of photocurable composition | | Average particle size (nm) | Comparative Example 1 | Comparative Example 2 | Example 1 | Example 2 | Example 3 | Example 4 | Example 5 |
|---|---|---|---|---|---|---|---|---|---|
| Evaluation of thickness accuracy | z-direction | z-direction thickness (mm) | 1.50 | 0.90 | 1.00 | 1.07 | 1.08 | 0.90 | 0.90 |
| | | Deviation from design value (%) | +50 | -10 | 0 | +7 | +8 | -10 | -10 |
| | | Evaluation result | C | A | A | A | A | A | A |
| | x-direction | x-direction thickness (mm) | 1.71 | 2.30 | 1.72 | 1.68 | 1.67 | 1.65 | 1.91 |
| | | Deviation from design value (%) | +7 | +44 | +8 | +5 | +4 | +3 | +19 |
| | | Evaluation result | A | C | A | A | A | A | B |

As shown in Table 1, the photocurable compositions of Examples 1 to 5, whose test pieces A1 had an X-ray absorption coefficient of from 9.0 $cm^{-1}$ to 34.0 $cm^{-1}$, were excellent in both the thickness accuracy in the z-direction (i.e., the propagation direction of light in the photomodeling) and the thickness accuracy in the x-direction (i.e., the direction perpendicular to the propagation direction of light in the photomodeling). This is believed to be because excessive and insufficient optical transparency of the photocurable composition were inhibited.

On the other hand, the photocurable composition of Comparative Example 1, whose test piece A1 had an X-ray absorption coefficient of less than 9.0 $cm^{-1}$, had an insufficient thickness accuracy in the z-direction (specifically, the z-direction thickness was excessively large). This is believed to be because, due to an excessively high optical transparency of the photocurable composition, even those unwanted portions that should not naturally be cured were cured in the z-direction during the photomodeling.

In addition, the photocurable composition of Comparative Example 2, whose test piece A1 had an X-ray absorption coefficient of more than 34.0 $cm^{-1}$, had an insufficient thickness accuracy in the x-direction (specifically, the x-direction thickness was excessively large). This is believed to be because, due to an insufficient optical transparency of the photocurable composition, the light used in the photomodeling was scattered in directions different from the natural propagation direction.

The disclosure of Japanese Patent Application No. 2020-056595 filed on March 26, 2020 is hereby incorporated by reference in its entirety.

All the documents, patent applications, and technical standards that are described in the present specification are hereby incorporated by reference to the same extent as if each individual document, patent application, or technical standard is concretely and individually described to be incorporated by reference.

## Claims

1. A photocurable composition, comprising a photopolymerizable component and a photopolymerization initiator, wherein:
in a case in which a rectangular sheet-like test piece A1 with a length of 40 mm, a width of 10 mm, and a thickness of 1 mm, is produced by photomodeling under conditions in which the photocurable composition is irradiated with visible light having a wavelength of 405 nm at an irradiation dose of 12 $mJ/cm^2$ to form a cured layer A1 with a thickness of 50 $\mu m$, the cured layer A1 is stacked in a thickness direction thereof to form a rectangular sheet-like modeling product A1 with a length of 40 mm, a width of 10 mm, and a thickness of 1 mm, and the modeling product A1 is irradiated with ultraviolet rays having a wavelength of 365 nm at an irradiation dose of 10 $J/cm^2$ to produce the test piece A1, the test piece A1 has an X-ray absorption coefficient of from 9.0 $cm^{-1}$ to 34.0 $cm^{-1}$.

2. The photocurable composition according to claim 1, further comprising a filler.

3. The photocurable composition according to claim 2, wherein the filler is at least one selected from the group consisting of silica particles, zirconia particles, aluminosilicate particles, alumina particles, and titania particles.

4. The photocurable composition according to claim 2 or 3, wherein the filler has an average particle size of from 5 nm to 200 nm.

5. The photocurable composition according to any one of claims 1 to 4, wherein:
in a case in which a discoid test piece A2 with a diameter of 15 mm and a thickness of 1 mm, is produced by photomodeling under conditions in which the photocurable composition is irradiated with visible light having a wavelength of 405 nm at an irradiation dose of 12 $mJ/cm^2$ to form a cured layer A2 with a thickness of 100 $\mu m$, the cured layer A2 is stacked in a thickness direction thereof to form a discoid modeling product A2 with a diameter of 15 mm and a thickness of 1 mm, and the modeling product A2 is irradiated with ultraviolet rays having a wavelength of 365 nm at an irradiation dose of 10 $J/cm^2$ to produce the test piece A2, the test piece A2 has a Vickers hardness of 18 HV or more.

6. The photocurable composition according to any one of claims 1 to 5, wherein:
in a case in which a rectangular rod-like test piece A3 with a length of 25 mm, a width of 2 mm, and a thickness of 2 mm, is produced by photomodeling under conditions in which the photocurable composition is irradiated with visible light having a wavelength of 405 nm at an irradiation dose of 12 $mJ/cm^2$ to form a cured layer A3 with a thickness of 100 $\mu m$, the cured layer A3 is stacked in a thickness direction thereof to form a rectangular rod-like modeling product A3 with a length of 25 mm, a width of 2 mm, and a thickness of 2 mm, and the modeling product A3 is irradiated with ultraviolet rays having a wavelength of 365 nm at an irradiation dose of 10 $J/cm^2$ to produce

the test piece A3, the test piece A3 has a bending elastic modulus of 3,000 MPa or more.

**7.** The photocurable composition according to any one of claims 1 to 6, wherein the photopolymerizable component comprises a (meth)acrylic monomer.

**8.** The photocurable composition according to claim 7, wherein:

the (meth)acrylic monomer comprises at least one of a monofunctional (meth)acrylic monomer or a bifunctional (meth)acrylic monomer, and
a total amount of the monofunctional (meth)acrylic monomer and the bifunctional (meth)acrylic monomer is not less than 90% by mass with respect to a total amount of the (meth)acrylic monomer.

**9.** The photocurable composition according to claim 7 or 8, wherein the (meth)acrylic monomer comprises a bifunctional (meth)acrylic monomer.

**10.** The photocurable composition according to any one of claims 1 to 9, which is a photocurable composition for photomodeling.

**11.** The photocurable composition according to any one of claims 1 to 10, which is used for the production of a dental product by photomodeling.

**12.** A three-dimensional modeling product, which is a cured product of the photocurable composition according to any one of claims 1 to 11.

**13.** A dental product, comprising the three-dimensional modeling product according to claim 12.

FIG.1

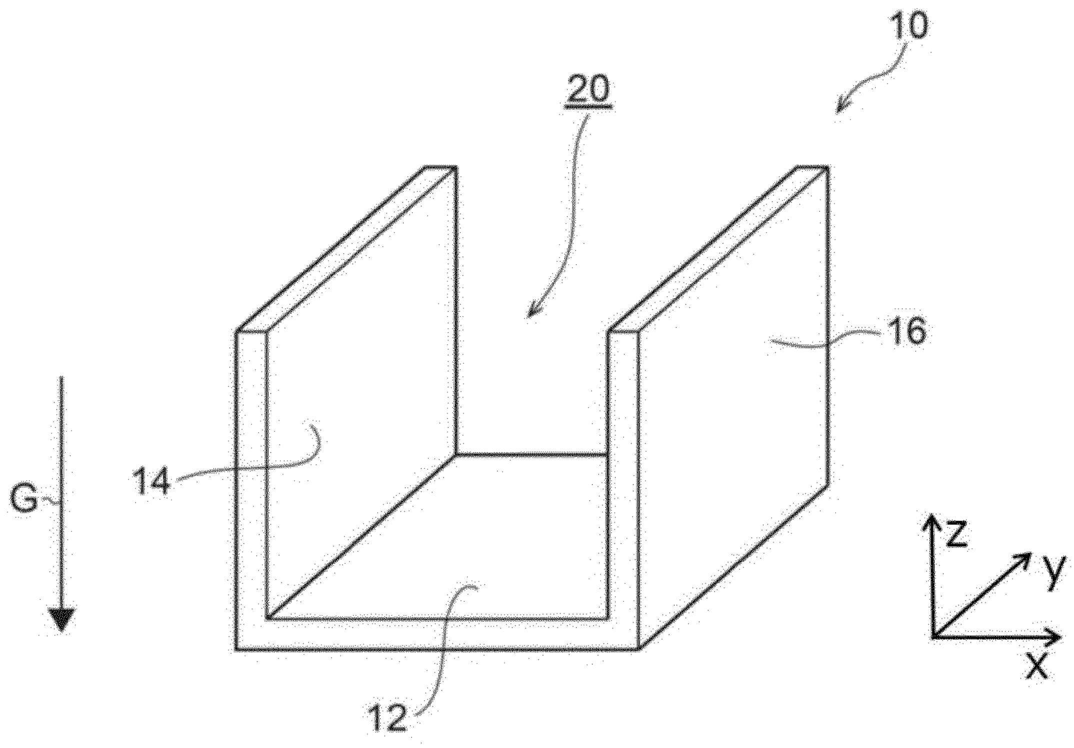

**INTERNATIONAL SEARCH REPORT**

International application No. PCT/JP2021/013083

A. CLASSIFICATION OF SUBJECT MATTER
Int.Cl. C08F2/46(2006.01)i, B33Y80/00(2015.01)i, B29C64/129(2017.01)i, B29C64/314(2017.01)i, A61K6/15(2020.01)i, B33Y70/00(2020.01)i
FI: B29C64/314, B29C64/129, A61K6/15, C08F2/46, B33Y70/00, B33Y80/00
According to International Patent Classification (IPC) or to both national classification and IPC

B. FIELDS SEARCHED
Minimum documentation searched (classification system followed by classification symbols)
Int.Cl. C08F2/46, B33Y80/00, B29C64/129, B29C64/314, A61R6/15, B33Y70/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2009-513764 A (HUNTSMAN ADVANCED MATERIALS (SWITZERLAND) GMBH) 02 April 2009 (2009-04-02), claims, paragraphs [0014]-[0186] | 1-13 |
| X | JP 2016-525150 A (DENTCA, INC.) 22 August 2016 (2016-08-22), claims, paragraphs [0011]-[0051] | 1-13 |
| A | WO 2019/074015 A1 (KURARAY NORITAKE DENTAL INC.) 18 April 2019 (2019-04-18), entire text | 1-13 |
| A | WO 2019/054507 A1 (TOKUYAMA DENTAL CORPORATION) 21 March 2019 (2019-03-21), entire text | 1-13 |
| A | JP 2007-126417 A (TOKUYAMA CORPORATION) 24 May 2007 (2007-05-24), entire text | 1-13 |
| A | WO 2019/048963 A1 (3M INNOVATIVE PROPERTIES COMPANY) 14 March 2019 (2019-03-14), entire text, all drawings | 1-13 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 April 2021 | 18 May 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/013083

C (Continuation). DOCUMENTS CONSIDERED TO BE RELEVANT

| Category* | Citation of document, with indication, where appropriate, of the relevant passages | Relevant to claim No. |
|---|---|---|
| P, A | US 2020/0172747 A1 (IVOCLAR VIVADENT AG) 04 June 2020 (2020-06-04), entire text | 1-13 |

Form PCT/ISA/210 (second sheet) (January 2015)

**INTERNATIONAL SEARCH REPORT**
Information on patent family members

International application No.
PCT/JP2021/013083

| | | |
|---|---|---|
| JP 2009-513764 A | 02 April 2009 | US 2010/0015408 A1<br>claims, paragraphs [0017]-[0189]<br>WO 2007/048819 A1<br>EP 1941322 A1<br>CN 101300527 A<br>KR 10-2008-0061383 A |
| JP 2016-525150 A | 22 August 2016 | US 2014/0239527 A1<br>claims, paragraphs [0011]-[0050]<br>WO 2014/172716 A1<br>EP 2986654 A1<br>CN 105246929 A<br>KR 10-2016-0055727 A |
| WO 2019/074015 A1 | 18 April 2019 | US 2020/0392272 A1<br>entire text<br>EP 3696198 A1 |
| WO 2019/054507 A1 | 21 March 2019 | US 2020/0253835 A1<br>entire text<br>EP 3682862 A1<br>CN 111093594 A<br>KR 10-2020-0054187 A |
| JP 2007-126417 A | 24 May 2007 | (Family: none) |
| WO 2019/048963 A1 | 14 March 2019 | US 2020/0197138 A1<br>EP 3681434 A1<br>CN 111050694 A<br>KR 10-2020-0051623 A |
| US 2020/0172747 A1 | 04 June 2020 | EP 3660087 A1 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4160311 B
- WO 2019189652 A
- JP 2020056595 A